# EUROPEAN PATENT APPLICATION

(11) **EP 4 725 542 A1**
(43) Date of publication of application: **15.04.2026**
(21) Application number: 24807637.4
(22) Date of filing: 17.05.2024
(51) Int. Cl.: A61N 1/05, A61N 1/36, A61N 1/08, A61B 5/00, A61B 5/1455

(54) **DIGITAL CORRECTION DEVICE**

(30) Priority: 18.05.2023 KR 20230064614; 18.05.2023 KR 20230064615; 09.06.2023 KR 20230074149; 22.03.2024 KR 20240040010
(71) Applicant: Rho, Hyung Tay, Seoul 07333 (KR)
(72) Inventor: Rho, Hyung Tay, Seoul 07333 (KR)
(74) Representative: RGTH
(86) International application number: PCT/KR2024/095810
(87) International publication number: WO 2024/237767

(57) **Abstract**

A digital corrective device according to an embodiment includes: a main body wearable inside a user's body; a sensing unit inserted into the main body and disposed at the targeted muscle of the user; an electrode unit disposed in the main body and applying a stimulation signal by contacting the target position of the user; and a control chip monitoring the state of the targeted muscle of the user in real-time from the sensing unit and generating the stimulation signal to change the sensory signal of the target position; wherein brainstem learning is induced by the sensory signal changed according to the stimulation signal, and a digital corrective device in which the movement of the targeted muscle for a specific case is corrected is provided.

## Description

### [Technical Field]

The present invention relates to a digital corrective device, and more specifically, to a system and the digital corrective device for diagnosing a specific case of a user, providing a correction method suitable for the user by utilizing a portable digital corrective device, and correcting the case by inducing brainstem learning of the user through the digital corrective device.

### [Background Art]

Generally, unconscious habits, that is, spontaneous habitual muscle movements, are caused by congenital factors and acquired factors such as stress, behavioral habits, eating habits, breathing methods, posture, and exercise habits.

Symptoms caused by spontaneous habitual muscle movements of a specific muscle are treated with drug correction when the symptoms are severe, and non-narcotic analgesics, muscle relaxants, tranquilizers, and antidepressants may be prescribed. In addition, Botox injection, which relieves muscle tension, is helpful in cases of severe muscle pain or muscle spasms

Besides that, appropriate physical correction and exercise therapy are proposed, or behavioral therapy is proposed.

Furthermore, through biofeedback, when a muscle tenses, a signal regarding this is transmitted to the user, allowing the user to train themselves to maintain a state of muscle relaxation.

However, correction using Botox is effective for about 3 months and must be newly administered every 3 months, and correction using muscle relaxants requires caution as dependency increases, and there is concern about side effects.

In the case of physical correction using low-frequency/highfrequency/ultrasound/infrared/laser, etc., it is just massaging the corresponding part and merely produces a temporary effect. Correction using exercise therapy merely relaxes the corresponding part and reduces fatigue, resulting in only a temporary effect.

Behavioral therapy and biofeedback correction are based on the hypothesis that the patient can change behavior by themselves. However, mastication, clenching, tongue position during sleep, tongue position during pronunciation, etc., are in most cases unconscious behaviors autonomously controlled by the brainstem, not conscious behaviors controlled by one's own will.

For example, when hard food enters, the brainstem automatically increases the chewing force to crush the food. It is the same principle that one unconsciously chews a small stone strongly when it is mixed in food. Also, when one is severely stressed or very angry, one unconsciously clenches their teeth. That is, therefore, instead of ordering the user to consciously control the corresponding muscle, the central nervous system must be made to control the corresponding muscle movement by itself.

### [Disclosure]

### [Technical Problem]

The present invention is proposed to solve the above problems, and provides a system that designs a digital device easily wearable by a user, wears it for a long time to secure quantitative data about the user to diagnose a case, and provide a correction method for each user.

Furthermore, a technical problem of the present invention is to provide a digital corrective device and a correction method utilizing the same, which can increase or decrease the activity of a target muscle by performing brainstem learning by continuously and periodically stimulating a sensory nerve by the digital device.

And, it is to provide an all-in-one system that can receive sensing data obtained for a long time from the digital corrective device at a user terminal and a server, and provide diagnosis and a correction method to a medical practitioner terminal by analyzing the corresponding sensing data at the server.

And, it is to provide a digital corrective device that can perform spontaneous control of a target muscle through brainstem learning in real time by designing and providing various digital corrective devices differently for each case.

### [Technical Solution]

A digital corrective device according to an embodiment may provide a digital corrective device including: a main body wearable inside a user's body; a sensing unit inserted into the main body and disposed on the target muscle of the user; an electrode unit disposed in the main body and contacting the target position of the user to apply a stimulation signal; and a control chip that monitors the state of the user's target muscle from the sensing unit in real time and generates the stimulation signal to change a sensory signal of the target position; wherein brainstem learning is induced by the sensory signal changed according to the stimulation signal, and movement of the target muscle for a specific case is corrected.

The digital corrective device may be a pronunciation corrective device for pronunciation correction of the user.

The electrode unit and the sensing unit may be integrated.

The integrated electrode unit and the sensing unit may include a plurality of electrode nodes arranged in a matrix array.

The integrated electrode unit and the sensing unit may detect the electrode node contacting the tongue when the user speaks for a specific pronunciation, and generate sensing data.

The control chip may emit the stimulation signal to the electrode node at a target position that must contact the tongue during the specific pronunciation, among the plurality of electrode nodes, according to the sensing data.

The integrated electrode unit and the sensing unit may penetrate the main body and be exposed to both the upper surface and the lower surface of the main body.

The integrated electrode unit and the sensing unit may include a stimulation node and a sensing node, respectively, which are exposed on opposite sides for each of the electrode nodes.

The digital corrective device may be a snoring corrective device for snoring correction of the user.

The main body may include a first main body and a second main body manufactured by obtaining the user's impression body with polymer resin and personalizing it.

The first main body may be wearable on the user's maxilla in the oral cavity and may include a sensing unit disposed on the palate.

The sensing unit may include a plurality of distance sensors arranged from the front to the rear.

The second main body may be wearable on the user's mandible in the oral cavity, and the electrode unit attached to the tongue root muscle of the tongue may be disposed.

The control chip may emit the stimulation signal to the electrode unit according to the sensing data of the distance sensors, and induce brainstem learning so that the tongue moves toward the lips by movement of the tongue root muscle.

The digital corrective device may activate the user's masticatory muscles to induce activation of the brain.

The sensing unit includes at least one pressure sensor inserted into the main body and aligned with the user's tooth occlusal surface or disposed between the teeth, and the electrode unit may provide the stimulation signal that magnifies the sensory signal of the periodontal ligament nerve and the nerve around the gums to be greater than or equal to a reference value.

The digital corrective device may be a wake-up device that operates in conjunction with a vehicle system, is worn in the user's oral cavity according to the user's drowsy driving state, and transmits a stimulation signal for activation of the brain to fight off drowsiness.

The digital corrective device may be a brain activation device for preventing or alleviating Alzheimer's or dementia by transmitting a stimulation signal for activating the user's brain.

### [Effect]

According to an embodiment, by providing a digital device easily wearable by the user and securing quantitative data about the user while worn for a long time, accurate diagnosis and correction of a case is possible through the quantitative data.

Brainstem learning is performed by adjusting a sensory signal through electrical stimulation to a nerve using an electronic drug, so that the intensity of movement for the target muscle is controlled by itself, and symptoms of the case can be alleviated. That is, a continuous correction effect using the brainstem learning effect based on the control of the sensory signal can be obtained.

As one example, by providing a pronunciation corrective device, mispronunciation for each pronunciation is monitored, and a stimulation signal is applied to a correct position to induce correct pronunciation, thereby inducing movement of the tongue muscle to the correct position, teaching muscle movement for pronunciation, and making unconscious pronunciation correction possible.

As another example, by providing a snoring corrective device during sleep, the position of the tongue inducing snoring is sensed, and an electrical stimulation signal is applied to control the position of the tongue in real time, thereby inducing tongue muscle movement, teaching muscle movement that prevents snoring, and making unconscious snoring correction possible.

As yet another example, a habit of alleviating temporomandibular joint disease can be formed by lowering the activation of temporomandibular joint muscles and masticatory muscles, and brain activation for brain diseases such as Alzheimer's or dementia can be induced by increasing the activation of masticatory muscles. In this regard, it is applicable as a wake-up device that induces brain activation and performs drowsiness prevention during vehicle driving.

The all-in-one system for diagnosis and correction through monitoring of such a digital device and modeling of the corresponding data can induce a patient to actively participate in treatment by the digital device designed centered on the user who is the patient, and can share results and induce regular management through an application installed on a user terminal such as a smartphone.

### [Description of Drawings]

FIG. 1 is a configuration diagram of an AI-based diagnosis and correction system including a digital device of the present invention.
FIG. 2 is a block diagram of the AI-based diagnosis and correction system including the digital device of FIG. 1
FIG. 3 is a detailed configuration diagram of the AI-based server of FIG. 1.
FIG. 4 is a flowchart illustrating the operation of the server of FIG. 3.
FIG. 5a is a block diagram of a corrective device of the digital device according to an embodiment, and FIG. 5b is a conceptual diagram of an electrode unit of the corrective device of the digital device according to an embodiment.
FIG. 6 is an operational diagram of the AI-based diagnosis and correction system to which the digital device according to an embodiment is applied.
FIG. 7 is a configuration diagram illustrating deep learning modeling of the server utilizing sensing data.
FIG. 8 is a conceptual diagram illustrating a correction method of the digital device according to an embodiment.
FIG. 9a and FIG. 9b are waveform diagrams illustrating a stimulation signal according to a magnitude of a sensing signal of the digital device according to an embodiment.
FIGS. 10a to 10b variously illustrate configuration diagrams of a digital device according to an embodiment of the present invention.
FIG. 11a and FIG. 11b illustrate configuration diagrams of a digital device according to another embodiment of the present invention.
FIG. 12a and FIG. 12b illustrate a sensing signal of the digital device of FIG. 11a and FIG. 11b.
FIG. 13a and FIG. 13b illustrate configuration diagrams of a digital device according to yet another embodiment of the present invention.
FIG. 14 is a flowchart explaining an operation of utilizing the treatment device of FIG. 13a and FIG. 13b as a wake-up device.
FIG. 15 is a conceptual diagram explaining a process of operating in conjunction with a vehicle system of the wake-up device.

### [Best Mode]

Hereinafter, embodiments of the present invention will be described in detail with reference to the accompanying drawings so that those skilled in the art to which the present invention pertains can easily implement them. However, the present invention can be implemented in various different forms and is not limited to the embodiments described herein. And, in the drawings, parts irrelevant to the description have been omitted for the sake of clear description of the present invention, and similar drawing reference numerals have been attached to similar parts throughout the specification.

Throughout the specification, when a certain part is referred to as "comprising" a certain component, it means that it may further include other components rather than excluding other components, unless specifically stated to the contrary.

Furthermore, the terms "...unit," "...section," or "...module" described in the specification mean a unit that processes at least one function or operation, and this may be implemented as hardware, software, or a combination of hardware and software.

In the present specification, "transmitting or providing" may include not only directly transmitting or providing but also indirectly transmitting or providing through another device or by utilizing a bypass path.

In the present specification, an expression described in the singular can be interpreted as singular or plural, unless an explicit expression such as "one" or "single" is used.

In the present specification, the same drawing reference numerals refer to the same components regardless of the drawing, and "and/or" includes each and every combination of one or more of the mentioned components.

In the present specification, terms including ordinal numbers such as first, second, etc., may be used to describe various components, but the components are not limited by the terms. The terms are used only for the purpose of distinguishing one component from another component. For example, a first component could be named as a second component without departing from the scope of the present disclosure, and similarly, a second component could also be named as a first component.

In the flowcharts described with reference to the drawings in the present specification, the order of operations may be changed, several operations may be merged, any operation may be divided, and a specific operation may not be performed.

Hereinafter, a preferred embodiment according to the present invention will be described in detail with reference to the accompanying drawings.

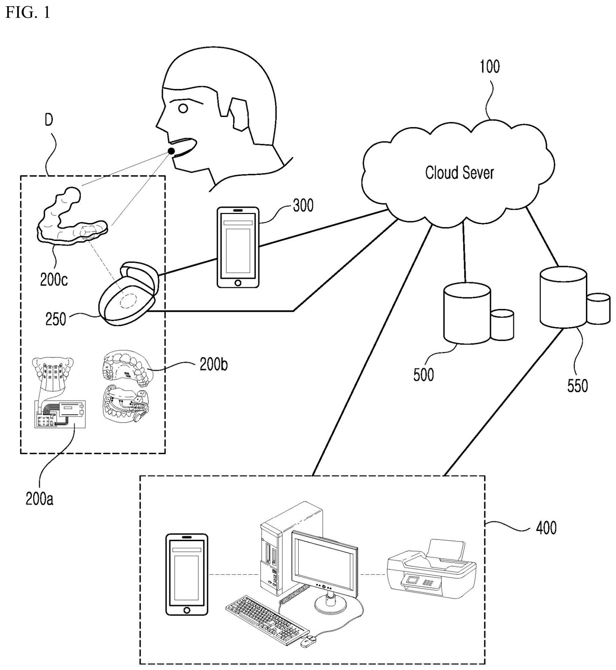

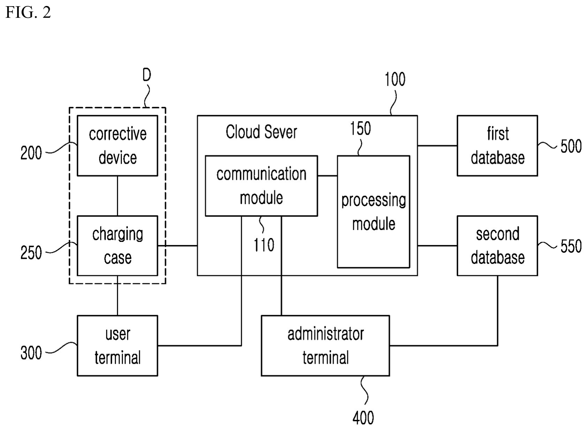

FIG. 1 is a configuration diagram of an AI-based diagnosis and correction system including a digital device (D) of the present invention, and FIG. 2 is a block diagram of the AI-based diagnosis and correction system including the digital device (D) of FIG. 1.

The AI-based diagnosis and correction system including the digital device (D) of the present invention includes a corrective device (200) as a digital device (D) wearable on the user's body, and includes a cloud server (100) for receiving the sensing data, processing the same, and calculating and proposing a case diagnosis and a correction method.

Furthermore, the AI-based diagnosis and correction system of the present invention includes the digital device (D), a user terminal (300), an administrator terminal (400), a first database (500), and a second database (550).

The digital device (D) includes a corrective device (200), and the corrective device (200) may be in a wireless form or a wired form worn on the body. The corrective device (200) may separately include a charging case (250), and may transmit sensing data through the corrective device (200) itself or the charging case (250). The user terminal (300) receives sensing data from the digital device (D), processes the same, and provides it to the user. The administrator terminal (400) receives a case diagnosis and a correction method from the cloud server (100). The first database (500) operates in conjunction with the cloud server (100) and performs the AI modeling. The second database (550) operates in conjunction with the administrator terminal (400) and stores medical data.

The corrective device (200) may be a detachable digital device equipped with a sensor specified according to the user's case, and may be a corrective device that includes an electrode unit capable of providing a stimulation signal for the corresponding case, and that can correct the case by providing a stimulation signal to a target position according to a user-specific correction method for the diagnosed case to induce movement of a target muscle.

The corrective device (200) stores sensing data generated by monitoring the user's state while being worn on a specific part of the user's body, for example, the oral cavity, abdomen, buttocks, arms, legs, etc., and can proceed with correction for the diagnosed case by continuously and periodically providing a stimulation signal to the user's target position according to the correction method. The user's state can be periodically monitored even while the correction is in progress, and if the correction method is supplemented according to the sensing data, the user's target position can be stimulated according to the supplemented stimulation signal. The corrective device (200) can be stored and charged in the charging case (250) after being detached from the user, and can transmit and receive sensing data and a control signal at the same time. Furthermore, the corrective device (200) can monitor the user's state in real time based on the calculated individual correction method of the user, and accordingly, change the stimulation signal and provide it to the user.

For this purpose, the corrective device (200) can perform near-field wireless communication with the charging case (250), and can transmit sensing data for a predetermined period at one time and receive a control signal, for example, through RF communication, etc.

Furthermore, it can be transmitted directly to the cloud server (100) wirelessly, or can be transmitted wirelessly to the administrator terminal (400).

*Meanwhile, the corrective device (200) can directly transmit sensing data generated by monitoring the user's state for a long time while being worn on the user, to the user terminal (300) by wire. It may include a transmission port for wired transmission, but is not limited thereto. Therefore, the corrective device (200) can transmit the sensing data, etc., both wirelessly and by wire, and data communication capable of receiving a control signal is possible.

Furthermore, the corrective device (200) may have a waterproofed IC chip inserted therein so that it can be worn for a long time, and power for driving a plurality of functional modules in the IC chip can be obtained through wired or wireless charging via the charging case (250).

The charging case (250) can be provided in the form of a general case (250), that is, a lower case (250) having a receiving part capable of receiving an object therein in an openable state, and a cover.

Furthermore, an IC chip may be inserted inside the charging case (250), and a form of a processor for wireless charging and data communication and processing can be provided.

The data communication may include at least two communication modules, but is not limited thereto.

As an example, the charging case (250) may include an RF receiving unit for receiving the sensing data through short-range wireless charging with the corrective device (200), and may further include a near-field wireless communication module for transmitting the sensing data to a nearby user terminal (300). In this case, the near-field wireless communication can proceed with wireless communication such as Bluetooth or BLE communicable by the user terminal (300), but is not limited thereto. A wire for wired transmission/reception of the sensing data may be further included, and transmission/reception with the charging case (250) and the corrective device (200) may be possible through various types of connectors (A-type, C-type, USB port, etc.).

The user terminal (300) is a terminal of the user wearing the corrective device (200), on which an application can be installed and executed, and can be implemented as a wireless communication capable module, for example, a smartphone, a tablet, a laptop, a PC, etc.

The user terminal (300) can download, install, and execute the corresponding case management application produced and distributed by the cloud server (100), thereby transmitting sensing data received from the charging case (250) to the cloud server (100) through the corresponding wireless communication.

Furthermore, the user terminal (300) can execute the case management application and periodically record the user's own status for managing the corresponding case, and this can proceed in the form of a medical questionnaire utilizing various entertainments.

Meanwhile, the administrator terminal (400) may be a device that receives case diagnoses, correction process records, and monitoring results for various users stored in the second database (550) from the cloud server (100), processes them so that they can be confirmed for medical treatment, and provides them to a medical practitioner, administrator, or counselor.

The administrator terminal (400) can be implemented as a mobile smartphone, tablet, laptop, PC, etc., like the user terminal (300), and may have a communication module that can operate in conjunction with the second database (550). Furthermore, a medical application that can operate in conjunction with the second database (550) and the cloud server (100) can be installed and executed on the administrator terminal (400).

The administrator application can be executed to communicate with the cloud server (100) and the user terminal (300) and utilized when diagnosing the case for the user, and the digital device (D) can be supplemented or the user's correction method can be calculated by reflecting it.

The cloud server (100) receives sensing data sensed for a long time from the digital device (D) from the user terminal (300), periodically receives medical questionnaire data of the user terminal (300), and processes it to diagnose a specific case specialized for the digital device (D) for the user, and calculate a user-specialized correction method.

The cloud server (100) classifies various sensing data and synthesizes it with information from various external databases or servers to perform diagnosis as to whether it corresponds to a specific case. At this time, the cloud server (100) includes an AI-based modeling engine for each case, and can perform diagnosis as to whether it corresponds to the specific case by driving each AI-based modeling engine. At this time, the cloud server (100) can implement modeling to enable diagnosis of the corresponding case by performing deep learning.

Furthermore, the cloud server (100) can transmit and receive necessary data by communicating with the user terminal (300) and the administrator terminal (400), and the generated result data can be stored in the second database (550). At this time, the first database (500) functions as a storage server unique to the cloud server (100), and the second database (550) is an administrator result database for the administrator terminal (400), which may be a separate cloud server for providing an administrator service, for example.

The cloud server (100) can generate, distribute, and manage various applications to provide the corresponding case correction service.

In this case, the various applications may mean different versions of applications according to the target.

Specifically, the user application synchronizes data with the charging case (250) and the cloud server (100) manually or automatically according to the setting of the patient, that is, the user. It can provide usage status such as target achievement rate and daily usage trend for continuous motivation of the user to use the digital device (D). Furthermore, the user application can display information such as an AI diagnosis index, a correction improvement effect, an expected correction period, and a correction process for a specific case calculated by the AI-based modeling engine from the cloud server (100). It can induce the user to perform the creation of a user-specific digital medical questionnaire according to the user's usage status and AI diagnosis result.

As an example, it may include a daily pain check, stress-related queries, and usage-related medical questionnaire. The patient's data is encrypted (for example, using the block cipher SEED) and transmitted after user authentication, and is encrypted and stored in the internal first database (500) configured with a firewall. The daily digital medical questionnaire allows recording of subjective health indicators such as important event records of the day, daily condition, and stress level, and this is provided with various entertaining effects to induce the user to maintain interest and responsiveness.

Meanwhile, the administrator application can input and query user information, specific sensing data, and diagnosis information for the correction of a specific user. Furthermore, the manufacturing of the related digital device (D) can be requested, and the management of the corresponding device can also be carried out.

The user's device usage status, sensing data measurement information, AI diagnosis result, digital medical questionnaire result, etc., can be provided as various statistical information to be utilized for correction. Verification and feedback from the administrator on the AI-based specific case diagnosis result can be received, and this can be transmitted to the cloud server (100). Furthermore, parameters such as the location of the stimulation, the intensity of the stimulation, and the cycle of the stimulation can be adjusted as specifications adjustment, wearing method, and correction method for the specific digital device (D), and individualized digital medical questionnaires can be optimized and provided according to the user's characteristics/symptoms.

The cloud server (100) may largely include a communication module (110) and a processing module (150), and the communication module (110) may perform communication for transmission/reception with the user terminal (300) and the administrator terminals (400) and communication with the first and second databases (500, 550).

The processing module (150) includes an AI-based diagnosis modeling engine (153) for each case, and can perform diagnosis as to whether it corresponds to the specific case by driving each AI-based diagnosis modeling engine (153). At this time, the processing module (150) can individually implement modeling to enable diagnosis of the corresponding case by performing deep learning for each case.

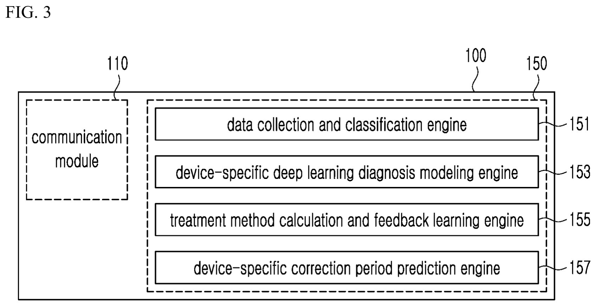

One example of the processing module (150) for this is shown in FIG. 3.

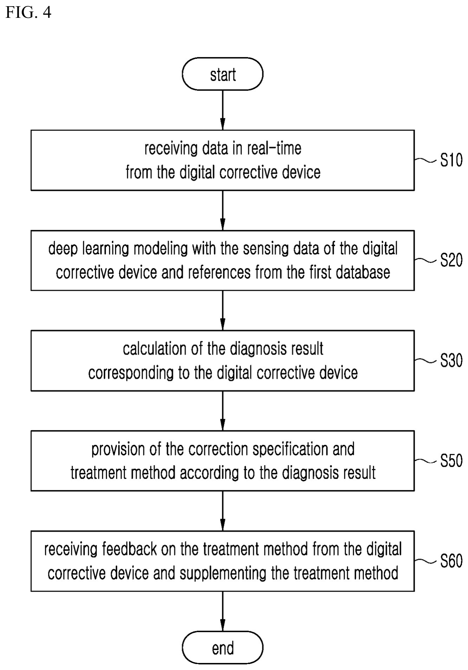

FIG. 3 is a detailed configuration diagram of the AI-based cloud server (100) of FIG. 1, and FIG. 4 is a flowchart illustrating the operation of the cloud server (100) of FIG. 3.

The processing module (150) of the cloud server (100) may be provided with different diagnosis modeling engines (153) for each case. The processing module (150) may primarily include a data collection and classification engine (151).

This is a pre-processing unit, which performs the task of classifying each obtained sensing data into corresponding case-specific data and sorting according to the same.

Therefore, through pre-processing, only data for the same case and for the same digital device (D) out of the large amount of sensing data obtained from a plurality of users, that is, a plurality of user terminals (300), can be trained with the same diagnosis modeling engine (153).

For one case, the collected engines can be largely classified into three, which can be distinguished as shown in FIG. 3, but are not limited thereto.

For each case, it may include a deep learning diagnosis modeling engine (153), a correction method calculation and feedback learning engine (155), and a user-specific correction period prediction engine (157).

Although each component is indicated as an engine, it can be classified as a unit or a module, and as a program, it can be understood as an individual algorithm or program.

The deep learning diagnosis modeling engine (153) can derive characteristic parameters for distinguishing between normal individuals and patients diagnosed with the corresponding case.

This may be different for each case; for example, in the case of pronunciation correction, the position of the tongue for each pronunciation can be defined as sensing data, and a part of the sensing data can be specified as characteristic parameters.

Next, these characteristic parameters are classified as singularities for distinguishing between normal individuals and users of the corresponding case while varying the sensing data in the time domain and the frequency domain, and an AI modeling that labels the corresponding characteristic parameter is generated by extracting the characteristic parameter.

Therefore, when the characteristic parameter is labeled in the sensing data injected through the AI modeling, a specific case is diagnosed based on this.

*As such, by training the labeled characteristic parameters, extracting significant patterns, and diagnosing, further reinforced AI modeling can be performed.

The AI modeling for each case may use CNN (Convolutional Neural Network) modeling, which is a deep learning modeling that extracts a spectrum and performs image processing in the time domain when extracting characteristic parameters.

Using CNN modeling, Feature Extraction is performed, which finds features in the converted spectrum image data and finds new features from the combination thereof, and in this case, various Data Augmentation methods and Model Regularization can be performed as reinforcement methods for exhibiting high performance even with incomplete and small data.

In particular, to supplement the quantity of initially collected data, by applying a transfer learning technique that solves the problem through fine-tuning based on a pre-trained model already trained on a large-scale dataset, modeling can be performed through fine-tuning based on a Resnet (Residual neural network) model where more than 1 million image data have already been trained, and as an example, Resnet18 can be applied.

As such, diagnosis for a specific case can be made through fine-tuned CNN modeling using transfer learning.

Meanwhile, the processing module (150) includes a correction method calculation and feedback learning engine (155).

The correction method calculation and feedback learning engine (155) can be developed to focus on the diagnosis result for a specific user.

The correction method calculation can provide various methods that can be proposed as a correction method for a specific case according to the diagnosis result of the specific user for the specific case.

Specifically, changes in the thickness, material, structure, position of the stimulation signal, intensity of the stimulation signal, cycle of the stimulation signal, etc., of the currently used corrective device (200) may be examples, and the wearing time of the corrective device (200) may also be one correction method.

The provision of such a correction method is provided to the medical practitioner terminal (400), and can be applied as data that can be additionally utilized for the diagnosis of the medical practitioner.

Meanwhile, the correction method calculation and feedback learning engine (155) analyzes the user's sensing data collected daily and the medical questionnaire data of the application from the user terminal (300), analyzes the patient's sensing data and digital medical questionnaire data, and provides feedback learning modeling that determines whether the correction is effectively progressing, thereby providing a more accurate supplemented correction method.

Such feedback learning modeling can be provided specialized for each user, and as sensing data and medical questionnaire data accumulate for each user, and sensing data is received as feedback on the correction method, it is labeled, and diagnosis result data from the medical practitioner terminal (400) obtained from directly treating the user is also utilized as a parameter.

At this time, by training the labeled sensing data, a correction method that is supplemented and strengthened based on the feedback can be calculated.

In addition, irregularity due to the user's voluntary use of the digital device (D) is set as a variable, and the inaccuracy of the digital medical questionnaire data regarding pain is also set as a variable, and a noisy-robust modeling can be performed by trying methods suitable for the data and the problem, such as Time Series Analysis, Anomaly Detection, Supervise-Learning, Similarity Deep Learning, or an Ensemble Model thereof.

The proposal of the correction method, which is the feedback result on such re-learning, is also transmitted to the administrator terminal (400), and feedback data on the corresponding correction method can be received according to the direct medical treatment of the user by experts.

Feedback data from the administrator terminal (400) can be received, supplemented, and re-learned.

Additionally, the processing module (150) may further include a correction period prediction engine (157) for each device.

The correction period prediction engine (157) for each device performs AI modeling that predicts the expected correction period based on the diagnosis data for the user and the sensing data of the user terminal (300) collected daily.

A Correction Cycle Vector (Life Cycle Vector) is extracted from the sensing data of several users who have already undergone correction with the corresponding digital device (D) and grouped, and the correction period is predicted by comparing the similarity with the Correction Cycle Vector (Life Cycle Vector) of the user to be predicted. At this time, the Correction Cycle Vector (Life Cycle Vector) can be extracted by creating a deep learning modeling that has learned the characteristics of the sensing data and the correction period.

The cloud server (100) of the AI-based diagnosis and correction system applying the digital corrective device (D) can have the configuration as shown in FIG. 3 and implement the operation as shown in FIG. 4.

Specifically, when sensing data for a predetermined time period from the corrective device (200), which is the digital device (D), is received along with the medical questionnaire data of the corresponding user terminal (300), medical treatment data for the user is received from the administrator terminal (400) (S10).

When the data collection is completed as such, the collected data, especially the sensing data, is input to the deep learning diagnosis modeling engine (153) for the corresponding corrective device (200), that is, the specific case, to extract characteristic parameters and perform modeling (S20).

As described above, a diagnosis result as to whether it corresponds to the case is calculated according to the synthesis of the characteristic parameters of the sensing data (S30).

At this time, the diagnosis result is verified by comparing the diagnosis result with the medical treatment data for the user, and additionally, the diagnosis result can be transmitted to the administrator terminal (400) to request verification (S40).

The cloud server (100) generates and provides a correction method that can correct the case corresponding to the diagnosis result in the correction method calculation and feedback learning engine (155) (S50).

The correction method aims for re-learning of the central nervous system so that a problematical habit can be controlled through brainstem learning of the user by providing a stimulation signal to a nerve at a target position of the user wearing the corrective device (200) and varying a sensory signal transmitted by the target nerve to the brainstem.

Specifically, by adjusting parameters such as the thickness, material, structure, stimulation signal position, stimulation signal intensity, and stimulation signal cycle of the currently used digital device (D), and providing a stimulation signal to a nerve at the target position, it can be learned to increase or decrease the activation level of a muscle causing a problematical habit, or change the movement and position of the muscle, by changing the corresponding sensory signal in the brainstem. Also, the wearing time of the digital device (D) may be one correction method.

Such brainstem learning is similar to the concept of Pavlovian conditioning, and can be defined as a process where the chain reaction process in which the target action of the target muscle occurs is analyzed, and the muscle is unconsciously controlled through a variable electrical signal and muscle control according to the analysis result, so that the target muscle spontaneously performs the target action.

The digital device (D) applies a stimulation signal to a sensory nerve at a target position according to each case, and the sensory signal varied by the stimulation signal is transmitted to the brainstem, so that learning progresses in the user's brainstem to enable the user to spontaneously or automatically adjust the intensity of movement of the target muscle, thereby inducing brainstem learning.

Such brainstem learning can be achieved when the stimulation signal is periodically applied to the nerve at the target position for a predetermined time or more, and if the wearing time is maintained for a predetermined time or more per single wear, and the wearing period is maintained for a predetermined period or more, the brainstem learning is completed even without wearing the digital device (D), i.e., without a stimulation signal, and the movement of the target muscle is spontaneously regulated, thereby exhibiting the effect of alleviating the case.

Specifically, when diagnosis for the user's case is completed from the cloud server (100) and a user-specific specialized correction method is proposed accordingly, stimulation signal information corresponding thereto may be provided. The stimulation signal information may include information on the target position of the stimulation signal for each user, i.e., the stimulation signal range which is position information for a specific electrode node among a plurality of electrode nodes, and the intensity of the stimulation signal, for example, the intensity of the current, and the cycle and duration time of the stimulation signal.

The digital device (D) according to the present invention can apply the stimulation signal to a target position specified according to the stimulation signal. The electrode unit can transmit the stimulation signal to the user's target position it is in contact with while flowing current.

Brainstem learning progresses by the sensory signal varied according to the stimulation signal as such, and movement of the target muscle is induced, and these movements of the target muscle correspond to the correction and symptom alleviation of the corresponding case. As the brainstem learning progresses, the spontaneity of the movement of the target muscle for correcting the corresponding case can be further strengthened. Accordingly, active and powerful brainstem learning can be achieved.

The user's target muscle may be at least one of an intraoral muscle, an arm muscle, a finger muscle, a foot muscle, a trunk (abdominal) muscle, a gluteal muscle, or a leg muscle.

The target muscle and the target position, that is, the target nerve, may be close or may coincide. In both cases where the target position (target nerve) and the target muscle coincide or are close, a plurality of electrode nodes for applying a stimulation signal can be attached to the corresponding target position.

Next, the user's sensing data is obtained daily from the user terminal (300) while wearing the corrective device (200) whose parameters have been changed according to the correction method.

Accordingly, feedback learning modeling that analyzes the user's sensing data and digital medical questionnaire data to determine whether the correction is effectively progressing is performed, and by continuously training and providing feedback on the sensing data for each user, the optimal correction method can be calculated (S60).

The proposal of the correction method, which is the feedback result on such re-learning, is also transmitted to the medical practitioner terminal (400), and feedback data on the corresponding correction method can be received according to the direct medical treatment of the user by expert medical practitioners. Feedback data from the medical practitioner terminal (400) can be received, supplemented, and re-learned.

As such, the cloud server (100) can receive feedback for each user until the point in time when the correction is finished, and provide an optimized correction method while supplementing the correction method.

As the AI cloud server (100), the user terminal (300), the corrective device (200), and the administrator terminal (400) transmit and receive data with each other, user monitoring for a long time at a long distance is possible between the user and the service provider.

Furthermore, when performing long-distance user monitoring, active intervention by the user is induced, and by transmitting and receiving data through various loops, optimal correction can be provided at the optimal time.

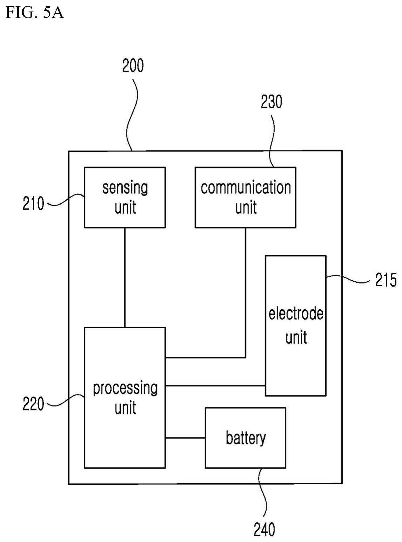

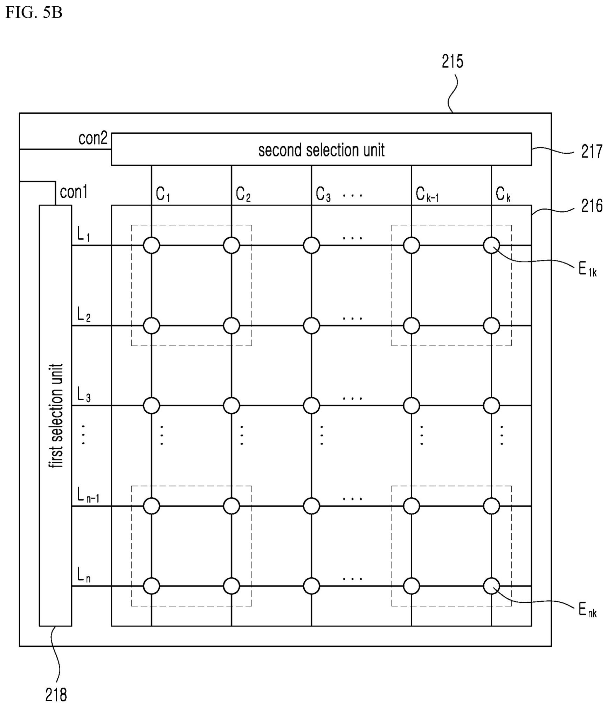

Hereinafter, a device for diagnosing and correcting a user's case and a system including the same will be described with reference to FIGS. 5 to 9.

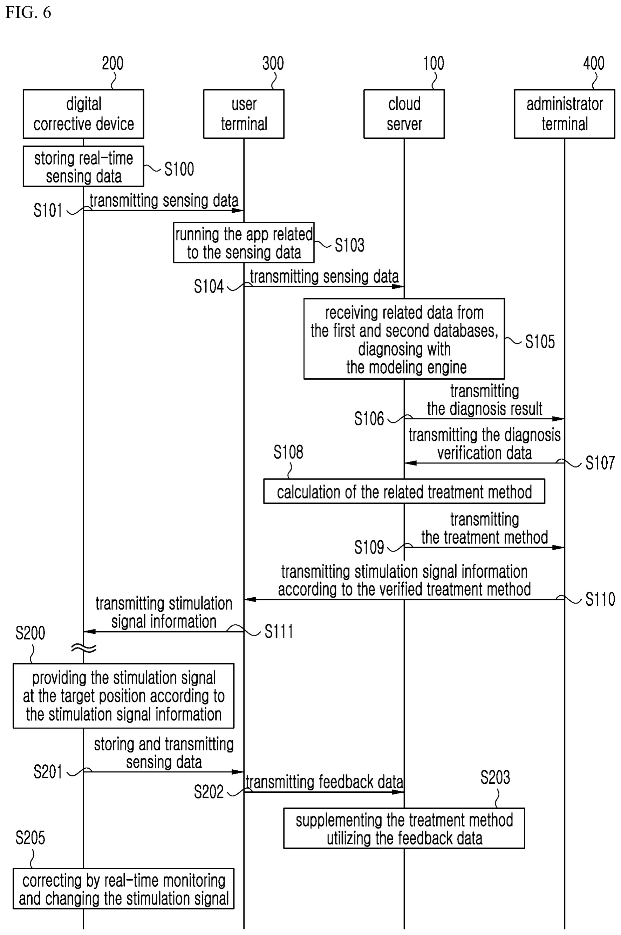

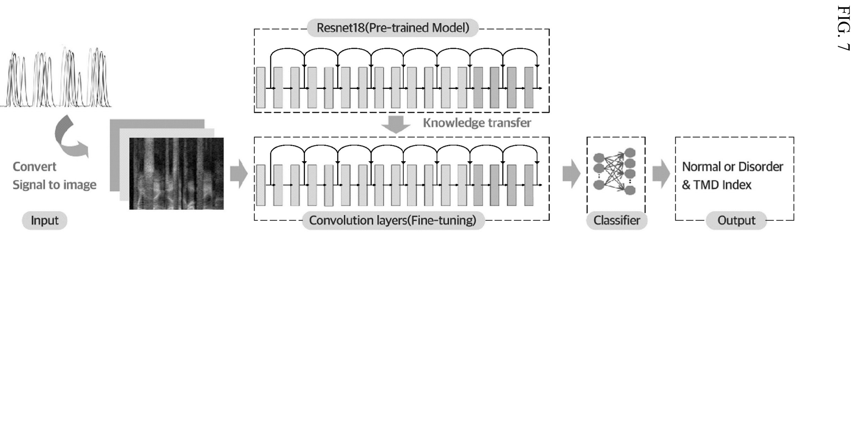

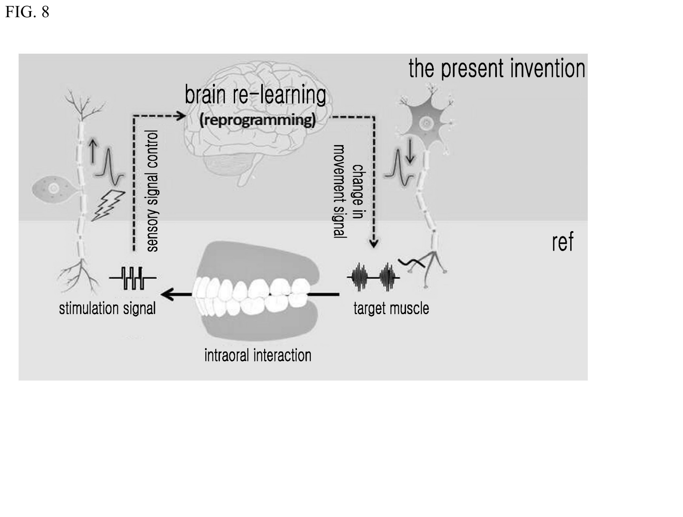

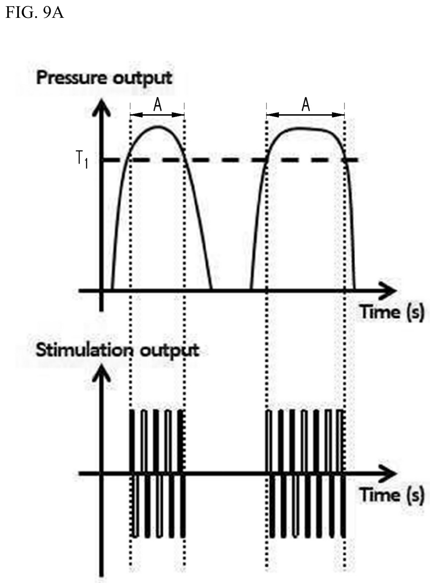

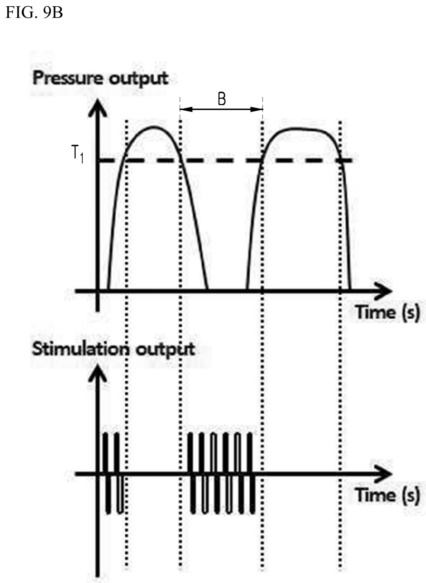

FIG. 5a is a block diagram of a corrective device according to an embodiment, FIG. 5b is a conceptual diagram of an electrode unit of the corrective device according to an embodiment, FIG. 6 is an operational diagram of the AI-based diagnosis and correction system to which the digital device according to an embodiment is applied, FIG. 7 is a configuration diagram illustrating deep learning modeling of a server utilizing sensing data, FIG. 8 is a conceptual diagram illustrating a correction method of the digital device according to an embodiment, and FIG. 9a and FIG. 9b are waveform diagrams illustrating a stimulation signal according to a magnitude of a sensing signal of the digital device according to an embodiment.

Referring to FIGS. 5a and 5b, the corrective device (200) of the digital device (D) according to the first embodiment may include a main body, and an electronic system integrated inside the main body, including a sensing unit (210) and an electrode unit

(215). A battery (240) may be included within the electronic system, but may be separately detached for balancing heat generation and weight, etc.

The electronic system may include a processing unit (220) and a communication unit (230) of FIG. 5a, but is not limited thereto.

The sensors of the sensing unit (210) are arranged on the main body to correspond to the user's target muscle and can sense the movement of the target muscle.

Therefore, sensing data due to habit is measured for a period of time while wearing the digital device (D).

The electrode unit (215) may be arranged on one surface of the main body. Specifically, the electrode unit (215) may be arranged at the user's target position to apply a stimulation along the sensory nerve at the target position.

Referring to FIG. 5b, as an output unit of the stimulation signal, the electrode unit (215) may include a plurality of electrode nodes (Enk) (216) arranged in rows and columns within a predetermined area. The electrode unit (215) may include a first selector (218) that selects a specific row from the plurality of electrode nodes (Enk) (216) and applies a first selection signal (L1-Ln), and a second selector (217) that selects a specific column and applies a second selection signal (C1-Ck).

A stimulation signal is emitted to at least one electrode node (Enk) (216) selected by the first selector (218) and the second selector (217), and the stimulation signal can be transmitted to the user's target position facing the electrode node (Enk) (216).

For this purpose, a plurality of first selection lines and second selection lines extending from the first selector (218) and the second selector (217) extend as a wire type, and the electrode node (Enk) (216) can be arranged at each intersection. The electrode unit (215) exposes the electrode node (Enk) (216) to the outside, and may include an insulating material that insulates the plurality of selection lines, and the insulating material may be made of an elastic insulating resin so that it can be attached to the user's body.

The stimulation signal can be simultaneously provided through the plurality of electrode nodes (Enk) (216), in which case, the first selection signal (L1-Ln) can be applied to the first selection lines of the plurality of first selectors (218), and the second selection signal (C1-Ck) can be applied to the second selection lines of the plurality of second selectors (217).

A common voltage may be applied as the first selection signal (L1-Ln), and the second selection signal (C1-Ck) is a stimulation signal, as a voltage of a specific intensity or a current signal of a specific intensity, and a predetermined current can flow due to the voltage difference of the corresponding signals in a specific electrode node (Enk) (216) where both selection signals (L1-Ln, C1-Ck) are applied, functioning as a resistive element.

As such, current can be transmitted to the sensory nerve at the target position it is in contact with by the current flowing through the specific electrode node (Enk) (216). The current can vary the sensory signal of the sensory nerve according to its current value and transmit it to the brainstem. Therefore, the variation of the sensory signal can be performed by adjusting the position and magnitude of the first selection signal (L1-Ln) and the second selection signal (C1-Ck).

As such, by applying a plurality of high-resolution electrode nodes (Enk) (216), the position of the sensory nerve at a different target position for each user can be finely adjusted to provide a stimulation signal to a specific position.

The first selection signal (L1-Ln) and the second selection signal (C1-Ck) as such, and the measured sensing data are stored in the processing unit (220), and can be transmitted through the user terminal (300) via the communication unit (230).

The corrective device (200) may further include a wireless charging unit, and the wireless charging unit is connected to the battery (240), and when the corrective device (200) is detached and placed inside the charging case (250), it can receive wireless power in conjunction with a wireless power transmitting unit on the bottom surface of the charging case (250).

The wireless power received as such can be stored in the battery (240) and utilized to operate the small electrical module of the digital corrective device (200), but unlike this, the battery (240) can also be charged through a wired connection.

The corrective device (200), as a wired or wireless digital device (D), can be worn on a target position of the user's body for a long time without a wire/with a wire connected to provide a stimulation signal to the user's body, and the user's state change according thereto can be sensed in real time for a long time, and the resulting sensing data can be stored and transmitted later, or the stimulation signal can be changed based on the real-time sensing data and provided to the target position. Alternatively, it can transmit and receive data with the case (250) through the communication unit (230).

The digital case (250) can be manufactured/sold/distributed together with the corrective device (200), but is not limited thereto.

The sensing data of the corrective device (200) can be directly transmitted to the user terminal (300), but can also be transmitted through the digital case (250).

Therefore, the digital charging case (250) is not absolutely necessary, but in the case where the corrective device is inserted into the oral cavity, considering that a separate case (250) is required for hygiene and cleaning, by adding a wireless charging function and an RF receiving function to the case (250), the functions of storage, charging, and data transmission/reception can be simultaneously implemented. However, the corrective device (200) may also transmit directly to the user terminal (300) via Bluetooth communication, or transmit to the user terminal (300) via wired communication through a wire, and this is not excluded.

Hereinafter, the operation of the AI-based diagnosis and correction system to which the corrective device (200) according to the embodiment is applied will be described with reference to FIGS. 6 to 9b.

Referring to FIG. 6, when the user maintains the digital device (D) worn on a target position on the body for a long time, for example, sleeps, the processing unit (220) obtains and records sensing data applied to the sensing unit (210) for a predetermined time (S100).

The state of the habitually targeted muscle can be determined according to the trend of the sensing data.

The sensing data during sleep as such can be transmitted to the user terminal by wireless communication or wired communication (S101).

Next, if an application for the user terminal (300) is installed and running on the user terminal (300), the user terminal (300) proceeds with the collection of the sensing data using near-field communication. Therefore, the sensing data is stored in the user terminal (300).

At this time, the user terminal (300) can periodically execute the application to induce the user to undergo a medical questionnaire (S103).

When the medical questionnaire as such is completed, both the sensing data and the medical questionnaire data are transmitted from the user terminal (300) to the cloud server (100) (S104).

When the cloud server (100) receives the sensing data and medical questionnaire data for the user, it receives related data from the first and second databases (500, 550), and performs diagnosis for a case corresponding to the specific digital device (D) with a related modeling engine (S105).

Specifically, the cloud server (100) performs deep learning by variously transforming the sensing data and extracting characteristic parameters. Furthermore, the cloud server (100) performs deep learning for diagnosis by receiving the corresponding user's medical treatment data provided from the second database (550) together.

The cloud server (100) variously transforms the received sensing data to extract characteristic parameters.

The process of extracting such characteristic parameters is, as previously described, capable of searching for characteristic parameters by transforming the corresponding input sensing data in the time domain (a) and the frequency domain (b) respectively, and capable of extracting characteristic parameters by classifying the sensing data.

The deep learning modeling in the cloud server (100), as shown in FIG. 7, transforms the input sensing data into an image through transformation and schematization of the sensing data when inputting the data into the corresponding modeling engine, and performs ground truth Labeling for deriving characteristic parameters from the image.

A classifier is utilized to train the labeled characteristic parameters, extract meaningful patterns, diagnose, and generalize.

At this time, the classifier can have Data Augmentation, Model Regularization, etc., applied, and before applying the classifier, pre-processing can be performed by applying a transfer learning technique that solves the problem through fine-tuning based on a pre-trained model trained on a large-scale dataset, in order to overcome the small amount of training data.

In particular, the Resnet model can be utilized, and as an example, after performing transfer learning with fine-tuning using Resnet18 (Residual neural network), which has been trained with more than 1 million image data, it is possible to proceed to the classifier. A case diagnosis result is derived through feature extraction in the classifier as such.

The cloud server (100) transmits the derived case diagnosis result to the first and second databases (500, 550) for storage, and transmits it to the administrator terminal (400) (S106).

The administrator terminal (400) can execute the administrator application to display the transmitted diagnosis result and provide it to the administrator.

The diagnosis result displayed by the application of the administrator terminal (400) can be provided along with the previous medical treatment record of the user. Specifically, the case diagnosis result can be displayed along with the user's previous X-ray image and history.

The administrator can perform verification of the case diagnosis through the administrator terminal (400).

That is, by comparing the diagnosis result with the user's direct monitoring data, verification can be performed on the validity of the diagnosis result of the cloud server (100) and transmitted (S107).

Next, the cloud server (100) can calculate a correction method related to the user's case based on the case diagnosis result (S108).

As a correction method, by performing unique modeling of the user and referring to the user's history, the optimal main body thickness, material, position of the user's target nerve, intensity of the stimulation signal according to the severity of the case, range of the stimulation signal, duration of the stimulation signal, and cycle of the stimulation signal for the user can be calculated and provided.

When the correction specification and the correction method are calculated, the corresponding correction method is transmitted to the administrator terminal (400) (S109).

The administrator terminal (400) can change the thickness and material of the main body in the corrective device (200) reflecting the correction method, adjust each parameter, and transmit this to the user terminal (300) as stimulation signal information (S110).

Furthermore, setting information including such stimulation signal information is also transmitted to the cloud server (100) and stored.

Meanwhile, the corrective device (200) may be remanufactured according to the reset data and worn by the user accordingly, or correction may proceed with variable wearing time and wearing conditions.

Specifically, stimulation signal information according to the verified correction method can be transmitted to the user terminal (300). The user terminal (300) sets the stimulation signal information in the application and can transmit the stimulation signal information to the corrective device (200) using near-field communication or wired communication (S111).

The verified correction method as such can provide a stimulation signal to perform brainstem learning according to sensory nerves at various target positions when diagnosing various cases and calculating correction methods.

The corrective device (200) receives the stimulation signal information and emits a stimulation signal to the target position accordingly (S200).

The corrective device (200) provides correction for the user's case.

Specifically, when diagnosis for a specific disease is completed from the cloud server (100) and a user-specific specialized correction method is proposed accordingly, stimulation signal information corresponding thereto may be provided. The stimulation signal information may include information on the target position of the stimulation signal for each user, i.e., the stimulation signal range which is position information for a specific electrode node (216) among a plurality of electrode nodes (216), or a specific node group, and the intensity of the stimulation signal, for example, the intensity of the current, and the cycle and duration time of the stimulation signal.

Reviewing FIG. 8, the corrective device (200) according to the present invention can apply the stimulation signal to a target position specified according to the stimulation signal. Such a stimulation signal can be converted and provided as control signals (con1, con2) for controlling the electrode unit (215) in the processing unit of FIGS. 5a and 5b.

The electrode unit (215) synchronizes the first selector (218) and the second selector (217) according to the received control signals (con1, con2) to provide the first selection signal (L1-Ln) and the second selection signal (C1-Ck) for activating the electrode node (216) at the corresponding position.

The selected electrode node (216) can transmit the stimulation signal to the user's target position it is in contact with while flowing current according to the first selection signal (L1-Ln) and the second selection signal (C1-Ck).

Such an operation is, for example, in the case where the corresponding case is temporomandibular joint disorder, the electrode unit (215) is arranged to be in contact with the lower part of the molar or the back part of the last molar, and a plurality of electrode nodes (216) are arranged in a matrix form. An electrode node (216) at a specific position is selected according to the stimulation signal information, and the corresponding electrode node (216) can correspond to a specific position of the user, the periodontal ligament nerve.

The position where the target nerves at the target position are concentrated may be different for each user, and the position of such target nerves can be supplemented by feedback from the corrective device (200).

The correction method as such is intended for the user to regulate the movement intensity and direction of the target muscle spontaneously or automatically by applying a stimulation signal to the target nerve.

That is, by applying a stimulation signal to the sensory nerve at the target position, the sensory signal injected into the brainstem is changed, and the movement of the target muscle in the brainstem can be induced to be performed in the correcting direction by the changed sensory signal.

Such brainstem learning can be achieved when the stimulation signal is periodically applied for a predetermined time or more, and if the wearing time is maintained for a predetermined time or more per single wear, and the wearing period is maintained for a predetermined period or more, the brainstem learning is completed even without wearing the corrective device (200), i.e., without a stimulation signal, so that the target muscle maintains a state of performing normal movement.

As shown in FIG. 8, the existing correction method (ref) for a specific case induced temporary muscle activation through medicine/physical correction, etc., rather than attempting to directly solve the problem of the central nervous system, which is the fundamental cause of the case. Alternatively, the existing correction method corrected the patient to consciously control the movement of the target muscle. However, such existing correction methods could not be a fundamental solution, although they might bring temporary effects.

In most cases, unconscious muscle movement autonomously regulated by the brainstem, rather than conscious action regulated by one's own will, can be autonomously and unconsciously modified by brainstem learning. That is, by regulating and injecting the sensory signal of the nerve at the target position into the brain, and the brain performing re-learning accordingly, the position of the target muscle can be changed, or the movement direction can be changed.

As an example, the corrective device (200) of the present invention can generate a movement signal for the target muscle through the regulation of sensory and noise intensity, as shown in (Section A) of FIG. 9a and (Section B) of FIG. 9b.

The electrode unit (215) can apply an electrical stimulation signal that is synchronized or anti-phasic sync to the sensing data to the target nerve. Specifically, as shown in FIG. 9a, the sensory signal is changed so that the sensory signal value of the target nerve is set to be slightly higher than or equal to a threshold value (T1). When the sensory signal to the nerve is increased to be greater than or equal to the threshold value (T1) as such, it exceeds the user's perception threshold of abnormality, and thus the intensity of the sensory signal accepted by the brain and the movement signal of the target muscle may be inversely proportional.

Therefore, by applying a stimulation signal so that the sensory data is set to be slightly higher than the threshold value at which the user feels discomfort, greater than or equal to the threshold value (T1), the user's brain is made to alleviate the target muscle.

Meanwhile, as shown in FIG. 9b, in the case where the sensory data is less than or equal to the threshold value (T1) (Section B), the proportionality between the sensory signal and the intensity of the movement of the target muscle can be utilized. That is, when the sensory data is less than or equal to the threshold value (T1), by applying noise as a stimulation signal, the signal-to-noise ratio becomes very large, and the sensory signal is relatively reduced, and accordingly, the brain can reduce the intensity of the movement. As such, by increasing the noise corresponding to the sensory data, the brain can alleviate the muscle activation level during the movement of the target muscle through learning.

The processing unit (220) can drive the electrode unit (215) to perform brainstem learning by applying one of the methods of increasing the sensory signal to be greater than or equal to the threshold value (T1), or increasing the noise while maintaining the sensory signal to be less than or equal to the threshold value (T1), according to the received stimulation signal information.

The electrode unit (215) trains the user's brain by periodically providing a stimulation signal to the target position for a predetermined period according to the stimulation signal information.

As the correction by the corrective device (200) proceeds for a long time, the corrective device (200) can periodically read and store the sensing data from the sensing unit (210) and transmit it to the user terminal (300) (S201).

The user terminal (300) receives the sensing data, and can generate feedback data by running the application and periodically performing a medical questionnaire. The medical questionnaire data can provide information such as daily correction progress, daily condition, and usage period. The user terminal (300) can transmit the feedback data to the cloud server (100) via the application (S202).

The cloud server (100) receives the feedback data, the medical questionnaire data, and the medical treatment data from the medical practitioner terminal (400) as such, and proceeds with deep learning training to supplement the correction method for the user (S203).

Accordingly, the correction method can be further strengthened, supplemented, and provided suitable for the user, and a repetitive feedback process is performed where the supplemented correction method is transmitted to the administrator terminal (400), verified, and then provided to the corrective device (200) and updated.

In addition, while the correction by the corrective device (200) proceeds for a long time, the corrective device (200) can read sensing data from the sensing unit (210) in real time, change the correction method based on this, and provide a changed stimulation signal to the target position (S205). As such, by the corrective device (200) itself modifying the correction method so that it can be slightly modified even in real time, the case can be corrected with a correction method further optimized for the user's current state.

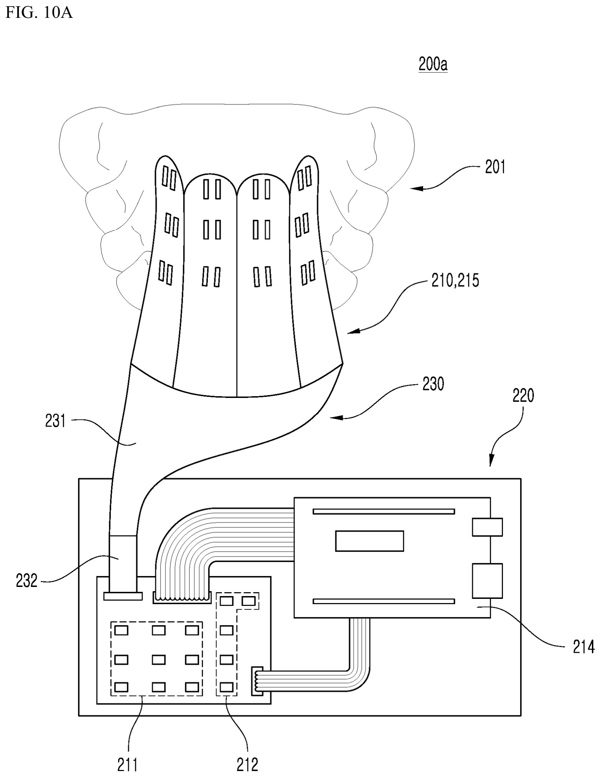

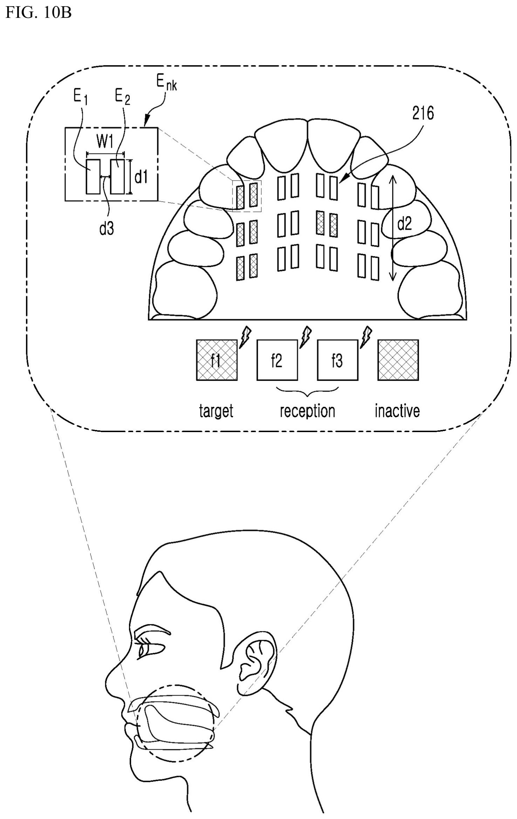

Hereinafter, a corrective device (200a) according to another embodiment of the present invention will be described with reference to FIGS. 10a and 10b. FIGS. 10a and 10b variously show configuration diagrams of a digital device according to an embodiment of the present invention.

Referring to FIGS. 10a and 10b, the corrective device (200a) according to another embodiment of the present invention may be a digital device (200a) for speech correction, and may have a configuration as shown in FIG. 10a.

Referring to FIG. 10a, the corrective device (200a) formed in the shape of an occlusal stabilization device (Splint) can prescribe the thickness and strength of the occlusal stabilization device inserted into the oral cavity based on the speech diagnosis and correction method, and can emit a stimulation signal of a predetermined magnitude to the user at the specific position from the electrode unit (215) it possesses, with a predetermined cycle according to the correction method.

The speech corrective device (200a) may include a splint body (201), an electrode unit integrated inside the body (201), and a communication unit (230) connected to the electrode unit. The splint body (201) is connected to one end of the communication unit (230), and the other end of the communication unit (230) may be connected to a processing module (220).

The processing module (220) is a processing unit implemented as a single chip (214), including an MCU, and can be implemented as a separate module connected to the electrode unit (210) of the body (201) through a connector (232) at the other end of the communication unit (230).

The processing module (220) may be connected via wiring to a circuit board on which the MCU, which is the processing unit (220), the stimulation unit array (211), and the impedance monitor (212) are arranged, but is not limited thereto. The MCU may be mounted on the circuit board and integrated.

When the processing module (220) is sufficiently miniaturized, it can be inserted into the splint body (201), receive a stimulation control signal applied to the electrode unit (215) wirelessly, and provide a stimulation signal to a specific position accordingly.

In the case of the speech corrective device, initial speech correction can be received by the cloud server (100) through a cross-user correction method, and initial setting can be made accordingly. In the case of the speech corrective device, in a state disconnected from an external system, it can autonomously monitor the user's speech state within the digital device (200a), and accordingly, induce tongue movement to the position according to the correction method to perform brainstem learning.

To this end, the electrode unit (215) and the stimulation unit (210) can be simultaneously implemented through the electrode array.

Specifically, the tongue's position information for a specific pronunciation can be received through the electrode unit (215). That is, when the tongue touches the electrode unit (215), movement information such as the tongue's contact position, contact intensity, and final distance during non-contact can be monitored.

That is, after requesting the user to vocalize a specific pronunciation, sensing data can be acquired by monitoring the tongue's movement information when the user vocalizes the specific pronunciation.

Through such real-time measurement, the impedance (resistance and capacitance) of specific electrodes in the electrode array changes, and the user's pronunciation state can be determined according to the position and amount of change of the changing electrodes.

Therefore, the processing module (220) can determine the user's pronunciation state through the sensing data, and accordingly modify the correction method to transmit an electrical stimulation signal to a specific position.

Specifically, as shown in FIG. 10b, the splint body (201) is produced as a personalized device based on an impression taken from the patient, that is, the user, at an institution, and is implemented as a double-layered splint body (201) using vacuum forming equipment to wrap the electronic components by applying acrylic resin or parylene coating to acrylic resin.

Therefore, it is implemented with a double surface so that it encloses the electronic components inside and is waterproofed to prevent moisture from entering from the outside, while being formed to wrap around and fix the teeth.

The splint body (201) includes a bottom surface that contacts the palate, and the electrode unit (215) may be arranged on the upper surface, which is the back side of the bottom surface, i.e., the surface facing the tongue.

The electrode nodes (216) of the electrode unit (215) maintain an exposed state to the outside of the body, and the space other than the electrode nodes (216) can be accommodated within the body (201) and maintain an insulated state.

Furthermore, the electrode nodes (216) penetrate the body (201) and are exposed on both the bottom surface that contacts the palate and the upper surface that is the back side of the bottom surface, and can function as both the electrode unit (215) and the stimulation unit (210).

Each of the electrode nodes (216) may include a plurality of electrode nodes (216) arranged in rows and columns within a predetermined area.

The predetermined area may be defined within the space formed by the width between the two molars corresponding to the distance ($d2$) between the user's first molar and canine on the palate, but is not limited thereto.

In FIG. 10b, two electrodes (216) are shown as being arranged in a pair at each node, but the two electrodes (216) of each node can operate simultaneously to obtain sensing data or provide a stimulation signal, but are not limited thereto.

That is, each electrode (216) (Enk) in each node can be driven individually, and according to an embodiment, one electrode (E1) can function as a sensing electrode and the other electrode (E2) can function as a stimulation electrode. When the electrodes (216) of each node perform different functions as such, the surfaces where the two electrodes (E1, E2) are exposed may be different from each other.

That is, in the case of the sensing electrode, which is one electrode (E1), it may be exposed facing the upper surface, and in the case of the stimulation electrode, which is the other electrode (E2), it may be exposed facing the bottom surface.

Each electrode (216) (Enk) in each node includes a predetermined separation distance (d3), has a first length (d1), is separated in the row direction, and may have a total width (w1) of the two electrodes (E1, E2) in the row direction. At this time, the first length (d1) may satisfy 3 to 5mm, preferably 4mm, and the total width (w1) may be equal to or smaller than the first length (d1). Preferably, it may satisfy 3.5mm to 4mm, but is not limited thereto.

Alternatively, when the two electrodes (E1, E2) of the electrode node (216) (Enk) function simultaneously, they are exposed to the outside of both surfaces, and may include an insulating material that insulates the plurality of selection lines, and the insulating material may be made of an elastic insulating resin so that it can be attached to the user's body.

The electrode unit (215) may include a first selector (218) that selects a specific row from the plurality of electrode nodes (216) (Enk) and applies a first selection signal (L1-Ln), and a second selector (217) that selects a specific column and applies a second selection signal (C1-Ck), which may be the same as in FIG. 5b.

A stimulation signal is emitted to at least one electrode node (216) (Enk) selected by the first selector (218) and the second selector (217), and when activating the electrode node (216) at the user's target position facing the electrode node (216) (Enk), for example, the electrode node (216) in row 2 and column 3, the first and second selection signals can be applied to activate the corresponding electrode node (216).

Therefore, the selected electrode node (216) is activated and a stimulation signal is applied, and accordingly, current can flow from the corresponding electrode node (216) to the target position on the palate. The current as such may correspond to a very small micro-current that does not cause harm to the human body.

The user perceives the target position by the current applied to the target position, moves the tongue to that position, and can pronounce the corresponding sound again.

Therefore, speech correction for a specific pronunciation is performed, and by repeating such speech correction, the user's tongue position can spontaneously move to the target position by brainstem learning when vocalizing the specific pronunciation.

To this end, a plurality of first selection lines and second selection lines extending from the first selector (218) and the second selector (217) extend as a wire type, and the electrode node (216) (Enk) can be arranged at each intersection.

The stimulation signal can be simultaneously provided through the plurality of electrode nodes (216) (Enk), in which case, the first selection signal (L1-Ln) can be applied to the first selection lines of the plurality of first selectors (218), and the second selection signal (C1-Ck) can be applied to the second selection lines of the plurality of second selectors (217).

A common voltage may be applied as the first selection signal (L1-Ln), and the second selection signal (C1-Ck) is a stimulation signal, as a voltage of a specific intensity or a current signal of a specific intensity, and a predetermined current can flow due to the voltage difference of the corresponding signals in a specific electrode node (216) (Enk) where both selection signals (L1-Ln, C1-Ck) are applied, functioning as a resistive element.

As such, current can be transmitted to the nerve at the target position on the palate that is in contact with, by the current flowing through the specific electrode node (216) (Enk). The current can vary the sensory signal of the nerve on the palate according to its current value and transmit it to the brainstem. Therefore, the variation of the sensory signal can be performed by adjusting the position and magnitude of the first selection signal (L1-Ln) and the second selection signal (C1-Ck).

As such, by applying a plurality of high-resolution electrode nodes (216) (Enk), the position of the palate nerve, which is different for each user, can be finely adjusted to provide a stimulation signal to a specific position.

*Furthermore, the current user's pronunciation state can be determined through monitoring of the tongue's movement during real-time pronunciation sensed by the sensing unit (210), and the intensity and area of the stimulation signal can be adjusted accordingly.

The first selection signal (L1-Ln) and the second selection signal (C1-Ck) as such, and the measured sensing data are stored in the processing module (220), and can be transmitted through the user terminal (300).

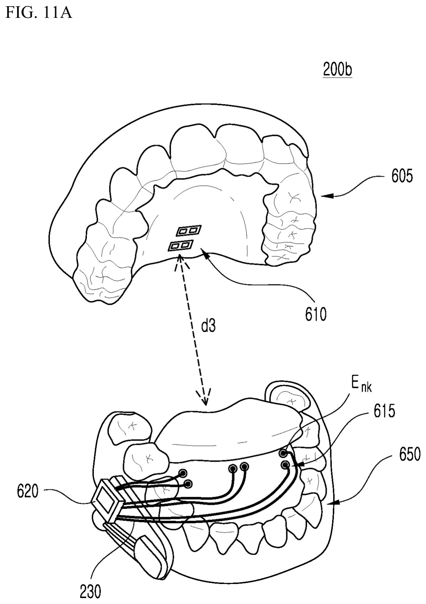

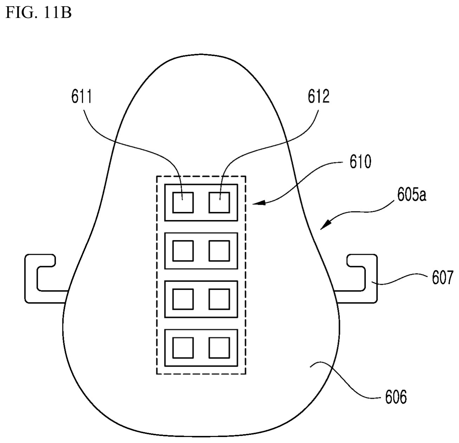

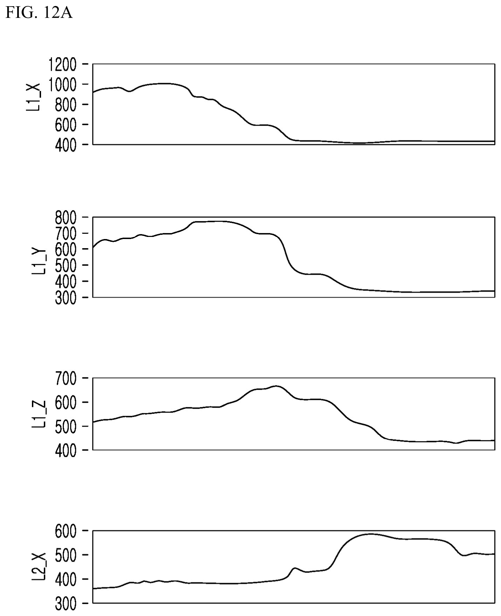

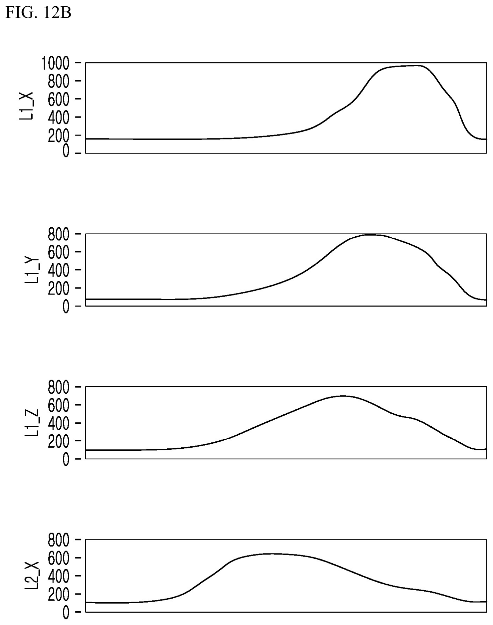

FIGS. 11a and 11b show configuration diagrams of a digital device according to yet another embodiment of the present invention, and FIG. 12 shows sensing signals of the digital device of FIGS. 11a and 11b.

Referring to FIGS. 11a and 11b, the corrective device (200b) according to yet another embodiment of the present invention may be a digital device (200b) for snoring correction, and may have a configuration as shown in FIG. 11a.

Referring to FIG. 11a, the corrective device (200b) formed in the shape of an occlusal stabilization device (Splint) includes two mutually separated occlusal stabilization devices (605, 650). Each occlusal stabilization device (605, 650) can prescribe the thickness and strength of the occlusal stabilization device (605, 650) based on the snoring diagnosis and correction method, and can respectively possess a sensing unit (610) and an electrode unit (615). Therefore, the user's current state can be monitored in real-time through the sensing unit (610), and based on that, the established treatment method can be processed to emit a stimulation signal of a predetermined magnitude to the user through the electrode unit (615) at a specific position with a predetermined cycle.

The snoring corrective device (200b) may include a first splint body (605), a second splint body (650), a sensing unit (610) integrated inside the first splint body (605), and a processing unit (620) connected to the sensing unit (610).

The second splint body (650) is arranged inside the user's maxilla, and an electrode unit (620) may be arranged inside, and the electrode unit (620) can also be connected to the processing unit (620).

The processing unit (620) may be formed as a single chip together with the communication unit, and may be inserted into one side of the first or second splint body (605, 650).

In FIG. 11a, it is shown as being inserted inside the second splint body (650), and accordingly, more signals can be transmitted and received to and from the electrode unit (620).

In the case of the snoring corrective device, initial snoring correction can be received by the cloud server (100) through a user-specific correction method, and initial setting can be made accordingly. In the case of the snoring corrective device, in a state disconnected from an external system, it can autonomously monitor the user's current state within the digital device, and accordingly, induce tongue movement to the position according to the correction method to perform brainstem learning.

To this end, information on the position of the tongue regarding the current state can be received through the sensing unit (610). That is, the sensing unit (610) may be a distance sensor, and can acquire sensing data by transmitting a sensing signal to the current tongue position and reading the distance to the current tongue according to the reflected signal.

Through such real-time measurement, the user's current tongue position can be determined, and accordingly, the correction method can be modified to transmit an electrical stimulation signal to a specific position through the electrode unit (615) attached to the user's genioglossus (hereinafter, GG muscle) to induce tongue movement.

Specifically, as shown in FIG. 11a, the first splint body (605) and the second splint body (650) are produced as personalized devices based on an impression taken from the patient, that is, the user, at an institution, and are implemented as double-layered splint bodies (605, 650) using vacuum forming equipment to wrap the electronic components by applying acrylic resin or parylene coating to acrylic resin.

Therefore, it is implemented with a double surface so that it encloses the electronic components inside and is waterproofed to prevent moisture from entering from the outside, while being formed to wrap around and fix the teeth.

The first splint body (605) includes a bottom surface that contacts the palate of the user's maxilla, and the sensing unit (610) may be arranged on the upper surface, which is the back side of the bottom surface, i.e., the surface facing the tongue.

At this time, the first splint body (605a), as shown in FIG. 11b, is provided in a form that can be attached to the palate, and can be implemented as the sensing unit (610) on the upper surface of the main body (606) of the first splint body (605a).

The sensing unit (610) may include a plurality of distance sensors, and each distance sensor may be arranged in a pair with a transmitter (611) and a receiver (612). Therefore, each distance sensor (611, 612) can calculate sensing data that sequentially varies according to the movement of the tongue.

As an example, FIGS. 12a and 12b show the output of the distance sensors at each position according to the movement of the tongue.

As shown in FIG. 12a, when the tongue moves from front to back, the four distance sensors (611, 612) can respectively observe the distance (d3) sequentially getting farther. Also, as shown in FIG. 12b, when the tongue moves from back to front, the four distance sensors (611, 612) can respectively observe the distance sequentially getting closer.

As such, the movement of the tongue can be accurately determined by monitoring the sensing data from the four distance sensors (611, 612) arranged anteroposteriorly on the palate together.

Meanwhile, the second splint body (650) can be worn on the user's mandible, and may include an electrode unit (615) that wraps around the teeth and is attached to the GG muscle under the tongue. The electrode unit (615) may include a plurality of electrode nodes (615) forming a row along the GG muscle, and can provide a stimulation signal to the plurality of electrode nodes (615) simultaneously or sequentially or with varying intensity.

The electrode nodes (615) of the electrode unit (615) maintain an exposed state to the outside of the body, and the space other than the electrode nodes (615) can be accommodated within the body (650) and maintain an insulated state.

Each of the electrode nodes (615) may include a plurality of electrode nodes (615) (Enk) arranged in rows and columns within a predetermined area.

In FIG. 11b, two electrode units (615) are shown as being arranged in a pair at each node, but the two electrodes (615) of each node can operate simultaneously to provide a stimulation signal, but are not limited thereto.

Alternatively, the electrode node (615) (Enk) is exposed to the outside on the surface that attaches to the GG muscle, and may include an insulating material that insulates the plurality of selection lines, and the insulating material may be made of an elastic insulating resin so that it can be attached to the user's body.

The electrode unit (615) emits a stimulation signal by selecting a specific row from the plurality of electrode nodes (615) (Enk), and activates the user's target position facing the electrode node (615) (Enk), and accordingly, current can flow from the corresponding electrode node (615) (Enk) to the target position of the GG muscle. The current as such may correspond to a very small micro-current that does not cause harm to the human body.

The user perceives the target position by the current applied to the target position, and brainstem learning proceeds by that stimulation current, which can induce the movement of the GG muscle to move the tongue toward the lips.

By repeatedly applying such a stimulation signal, the GG muscle moves the tongue toward the lips, so that brainstem learning can be achieved to prevent the tongue from unconsciously rolling back towards the back of the palate into the throat during sleep.

Information on the measured sensing data and the stimulation signal as such is stored in the processing module (620), and can be transmitted through the user terminal (300).

Although the digital corrective device (200a, 200b) has been described as being worn inside the oral cavity, as shown in FIGS. 10a to 12, it is not limited thereto, and it can be worn on any part of the user's body that requires correction of unconscious muscle movement, for example, the arms, legs, waist, abdominal muscles, etc.

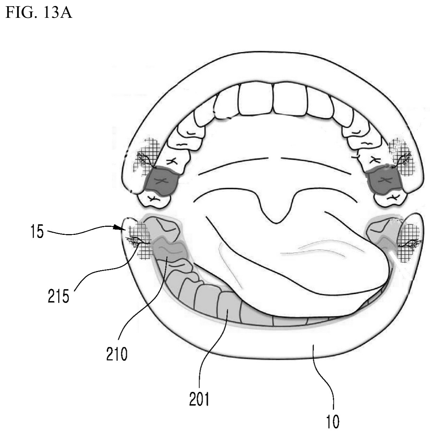

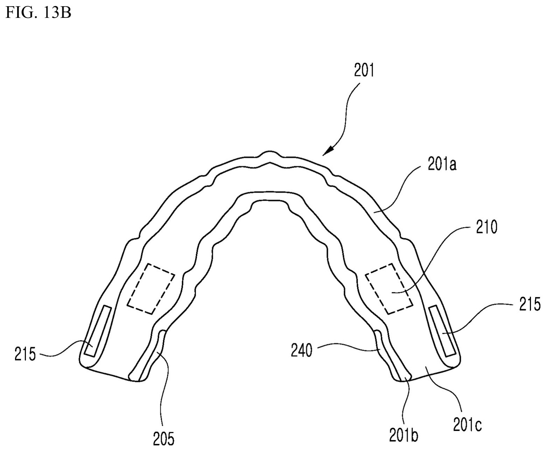

FIGS. 13a and 13b show configuration diagrams of a digital device according to yet another embodiment of the present invention.

FIGS. 13a and 13b show that the digital device (200c) according to yet another embodiment of the present invention can regulate the mastication intensity by changing the periodontal nerve sensory signal, which is a part of the various sensory signals applied to the brainstem controlling the jaw muscles, by adjusting the activation level of the jaw muscles.

By applying a stimulation signal to the periodontal ligament nerve, the sensory signal injected into the brainstem is changed, and due to the changed sensory signal, the brainstem will automatically lower or raise the activation level of the jaw muscles performing masticatory movement, allowing the treatment to proceed.

Such brainstem learning can be achieved when the stimulation signal is periodically applied to the nerve at the target position for a predetermined time or more, and if the wearing time is maintained for a predetermined time or more per single wear, and the wearing period is maintained for a predetermined period or more, the brainstem learning is completed even without wearing the treatment device (200), i.e., without a stimulation signal, so that specific muscle movement is automatically regulated and the effect of disease alleviation can be expressed.

The treatment device (200c) has the shape of an occlusal stabilization device (Splint), and can prescribe the thickness and strength of the occlusal stabilization device based on the diagnosis result and treatment method, and can emit a stimulation signal of a predetermined magnitude from a specific position of the electrode unit (215) it possesses to the user at the specific position with a predetermined cycle according to the treatment method. Additionally, it can be optimized to observe changes in inter-tooth pressure data and temporomandibular joint muscle activation level according to the wearing period and individual characteristics (based on daily digital medical questionnaire).

Referring to FIGS. 13a and 13b, the treatment device (200c) of the digital device (D) may include a splint body (201), an electronic system (205) integrated inside the body (201), a pressure sensor (210), and an electrode unit (215). At this time, the battery (240) may be included within the electronic system (205), but may be separately detached for the balance of heat generation and weight, etc.

The electronic system (205) may include the processing unit (220) and the communication unit (230) of FIG. 5a, but is not limited thereto.

The electronic system (205) is implemented as a single chip (205) and inserted on one side of the splint body (201), and the battery (240) is inserted on the opposite side, allowing wire connection to each other within the splint body (201).

The splint body (201) is produced as a personalized device based on an impression taken from the patient, that is, the user, at a hospital, and is implemented as a double-layered splint body (201) using vacuum forming equipment to wrap the electronic components by applying acrylic resin or parylene coating to acrylic resin.

Therefore, it is implemented with a double surface so that it encloses the electronic components inside and is waterproofed to prevent moisture from entering from the outside, while being formed to wrap around and fix the teeth (10).

Accordingly, the splint body (201) includes a first surface (201a) that contacts the outer side surface of the tooth (10), a second surface (201b) that contacts the inner side surface of the tooth (10), and an upper surface (201c) that contacts the upper surface of the tooth (10), and the upper surface (201c) may be bent between the first surface (201a) and the second surface (201b).

The electronic system (205) and the battery (240) may be accommodated within the second surface (201b), and pressure sensors (210) may be accommodated on the upper surface. Specifically, the sensor electrodes of the pressure sensor (210) are arranged within the upper surface (201c) of the splint body (201) so as to overlap the occlusal surface of the user's molar, thereby aligning with the molar in an insulated state.

Therefore, the inter-tooth pressure due to mastication or habit is measured for a predetermined time, for example, during sleeping time, while wearing the digital device (D).

The electrode unit (215) may be arranged inside the first surface (201a) of the splint body (201), facing the outer side surface of the tooth (10). Specifically, the electrode unit (215) may be arranged opposite the gingiva side of the molar so as to be able to apply stimulation along the periodontal ligament nerve toward the molar. The first surface (201a) may be formed to partially cover the gingiva so that the electrode unit (215) corresponds to the lower part of the tooth, that is, the periodontal ligament nerve, on the first surface (201a).

Alternatively, the electrode unit (215) may be arranged on the inner side of the second surface (201b) so as to correspond to the periodontal ligament nerve.

*The electrode unit (215) may include a plurality of electrode nodes (Enk) (216) arranged in rows and columns within a predetermined area, as shown in FIG. 5b, and may have the same configuration.

Current can be transmitted to the adjacent periodontal nerve by the current flowing through the specific electrode node (Enk) (216). The current can vary the sensory signal of the periodontal nerve according to its current value and allow it to be transmitted to the brainstem. Therefore, the variation of the sensory signal can be performed by adjusting the position and magnitude of each selection signal.

As such, by applying a plurality of high-resolution electrode nodes (Enk) (216), the position of the periodontal ligament nerve, which is different for each user, can be finely adjusted to provide a stimulation signal to a specific position.

The sensing data regarding the respective selection signals and the measured inter-tooth pressure as such is stored in the processing unit (220), and can be transmitted through the case (250) via the communication unit (230), or transmitted through the user terminal (300).

The treatment device (200c) is a wireless digital device (D) that can be worn by the user in the oral cavity for a long time without wires to provide a stimulation signal to the user's body, can sense changes in the user's state over a long period, and can store and subsequently transmit the resulting sensing data for a long time.

The sensing data of the treatment device (200c) may be directly transmittable to the user terminal (300), but can be transmitted through the digital case (250).

The treatment device (200c) receives sensing data in real-time, changes the treatment method according to the sensing data based on the treatment method, and can apply the stimulation signal to the periodontal ligament nerve at the target position. As such, a treatment method further adapted to the user can be provided by the active and autonomous real-time correction of the treatment method by the treatment device (200c) itself, and effective improvement of the disease is possible through brainstem learning.

As an example, if the specific disease is TMD, the treatment device (200c) can reduce the tension of the jaw muscles by weakening the activation of the jaw muscles and thus lowering the inter-tooth pressure. Therefore, masticatory habits that alleviate temporomandibular joint disease can be maintained.

On the other hand, if the specific disease is Alzheimer's or dementia, the treatment device (200c) can apply a stimulation signal in the direction of enhancing the activation of the jaw muscles. When a strong stimulation signal is applied to the periodontal ligament nerve and the surrounding gingival nerve, the masticatory muscles contract strongly, giving a large stimulus to the temporomandibular joint muscles. This stimulus can transmit a strong signal to the brain, thereby increasing brain activity. If such masticatory habits are formed, they stimulate the brain of Alzheimer's or dementia patients, alleviating the symptoms and potentially delaying the onset of these diseases, which are caused by reduced brain activity.

In fact, elderly people who have lost all their teeth show significantly faster progression of dementia, which is reported to occur due to the reduced brain activity resulting from unhindered mastication. As such, habituation aligned with the biological rhythm of mastication, which occurs very frequently in daily life, can greatly increase brain activity, thereby alleviating diseases expressed from the deactivation of the brain, such as dementia or Alzheimer's.

Meanwhile, the treatment device (200c) can be applied as a wake-up device. The treatment device (200c) can apply a stimulation signal to the periodontal ligament nerve and the surrounding gingival nerve in the direction of enhancing the activation of the jaw muscles. When the user performs mastication while wearing the treatment device (200c), the jaw muscles are activated by the stimulation signal, transmitting a strong signal to the brain, and thus waking up the user through brain activation.

Such a wake-up device can, in particular, be linked with a vehicle system to perform a function for immediate drowsiness countermeasures.

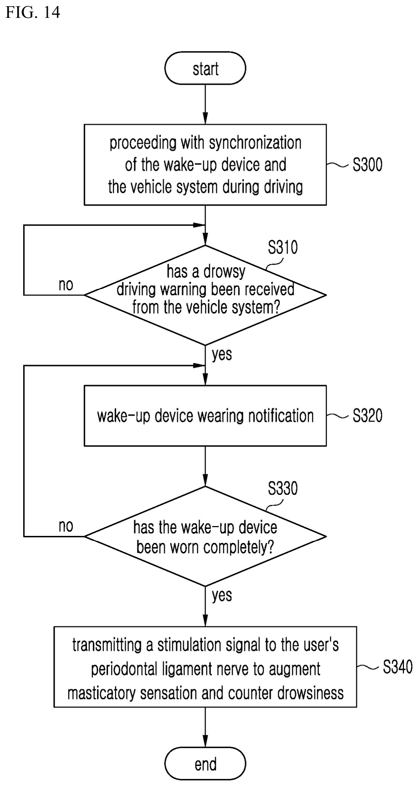

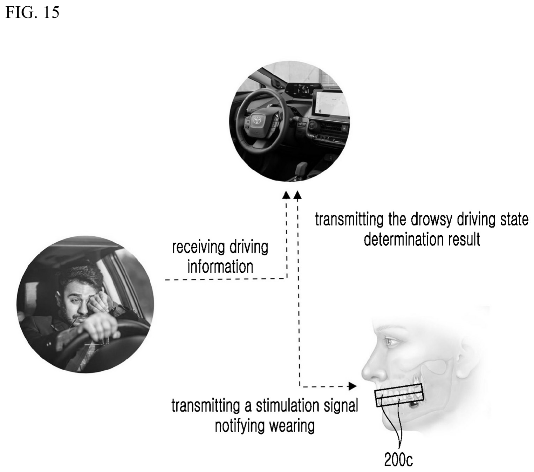

Hereinafter, the operation of the wake-up device will be described with reference to FIGS. 14 and 15.

FIG. 14 is a flowchart illustrating the operation of utilizing the treatment device of FIGS. 13a and 13b as a wake-up device, and FIG. 15 is a conceptual diagram illustrating the process of linking the wake-up device with a vehicle system.

Referring to FIGS. 14 and 15, the wake-up device (200c) is provided within the vehicle and linked with the vehicle system to prevent the user's drowsy driving.

Specifically, the wake-up device (200c) may have the same configuration as FIGS. 13a and 13b, and can be worn in the user's oral cavity to perform an action capable of waking up the user from drowsiness by transmitting a strong stimulus to the brain through the activation of the jaw muscles, including the masticatory muscles. At this time, the wake-up device (200c) is provided within the vehicle and can be linked with the vehicle system (600) (S300).

First, the wake-up device (200c), which is a digital device, may be stored in the case (250) and placed inside the vehicle. At this time, the wake-up device (200c) inside the vehicle and the vehicle system (600) can maintain a synchronized and communicable state, i.e., a Bluetooth connected state, and thus can transmit and receive signals.

While the user is driving the vehicle, the vehicle system (600) can periodically obtain the user's driving information to determine the user's driving state. That is, the vehicle system (600) can determine whether the user's driving state is a drowsy driving state by combining information such as road condition, driving direction, and speed change.

When the vehicle system (600) periodically determines the user's driving state and determines it to be a drowsy driving state, it can transmit the corresponding information to the linked wake-up device (200c) to issue a warning (S310).

When the wake-up device (200c) receives a warning about the drowsy driving state from the vehicle system (600), it can proceed with a wearing notification to the user (S320). The wearing notification can be transmitted to the user as voice data through the speaker of the vehicle system (600) by sending a receipt confirmation to the vehicle system (600). Alternatively, the wearing notification can be delivered to the user in various ways through the user interface of the wake-up device (200c) itself, such as vibration/light emission/voice.

When the user wears the wake-up device (200c) in the oral cavity according to the wearing notification (S330), the wearing notification is stopped, and a stimulation signal can be transmitted from the wake-up device (200c) to the user's target nerve (S340).

Specifically, sensory augmentation can be performed by transmitting a stimulation signal to the user's temporomandibular joint muscles and masticatory muscles to enhance the masticatory sensation for transmission to the brain. When the sensory signal for the augmented sensation is transmitted to the brain as such, the brain is activated by the strong sensory signal and can escape from the drowsy state.

As such, the wake-up device (200c) can include a function capable of immediately countering drowsiness through brain activation by being linked with the vehicle system (600), and can be diversely modified and applied.

Although the corrective device (200a, 200b, 200c) has been described as being worn inside the oral cavity, as shown in FIGS. 10a to 13b, it is not limited thereto, and it can be worn on any part of the user's body that requires correction of unconscious muscle movement, for example, the arms, legs, waist, abdominal muscles, etc.

Although the embodiments of the present invention have been described in detail above, the scope of the present invention is not limited thereto, and various modifications and improvements by those skilled in the art using the basic concept of the present invention as defined in the following claims also fall within the scope of the present invention.

## Claims

1. A digital corrective device, comprising: a main body wearable in the oral cavity of a user; a sensing unit inserted into the main body and arranged at the targeted muscle of the user; an electrode unit arranged in the main body and applying a stimulation signal by contacting the target position of the user; and a control chip monitoring the state of the targeted muscle of the user in real-time from the sensing unit and generating the stimulation signal to change the sensory signal of the target position; wherein brainstem learning is induced by the sensory signal changed according to the stimulation signal, and the movement of the targeted muscle for a specific case is corrected.

2. The digital corrective device according to Claim 1, wherein the digital corrective device is a pronunciation corrective device for the pronunciation correction of the user.

3. The digital corrective device according to Claim 1, wherein the main body is a device manufactured as a personalized device by acquiring the user's impression with a polymer resin.

4. The digital corrective device according to Claim 2, wherein the electrode unit and the sensing unit are integrated.

5. The digital corrective device according to Claim 4, wherein the integrated electrode unit and the sensing unit include a plurality of electrode nodes arranged in a matrix array.

6. The digital corrective device according to Claim 5, wherein the integrated electrode unit and the sensing unit generate sensing data by sensing the electrode nodes contacting the tongue during the user's vocalization for the specific pronunciation.

7. The digital corrective device according to Claim 6, wherein the control chip emits the stimulation signal to the electrode nodes at the target position that should contact the tongue during the specific pronunciation among the plurality of electrode nodes according to the sensing data.

8. The digital corrective device according to Claim 7, wherein the integrated electrode unit and the sensing unit penetrate the main body and are exposed to both the upper surface and the lower surface of the main body.

9. The digital corrective device according to Claim 7, wherein the integrated electrode unit and the sensing unit respectively include a stimulation node and a sensing node exposed on opposite surfaces for each of the electrode nodes.

10. The digital corrective device according to Claim 1, wherein the digital corrective device is a snoring corrective device for the snoring correction of the user.

11. The digital corrective device according to Claim 10, wherein the main body includes a first main body and a second main body manufactured as a personalized device by acquiring the user's impression body with a polymer resin.

12. The digital corrective device according to Claim 11, wherein the first main body is wearable in the maxilla in the oral cavity of the user, and includes the sensing unit arranged on the palate.

13. The digital corrective device according to Claim 12, wherein the sensing unit includes a plurality of distance sensors arranged from front to back.

14. The digital corrective device according to Claim 13, wherein the second main body is wearable in the mandible in the oral cavity of the user, and the electrode unit contacting the genioglossus of the tongue is arranged.

15. The digital corrective device according to Claim 14, wherein the control chip induces brainstem learning so that the tongue moves toward the lips by the movement of the genioglossus by emitting the stimulation signal to the electrode unit according to the sensing data of the distance sensors.

16. The digital corrective device according to Claim 1, wherein the digital corrective device is a digital corrective device that induces the activation of the brain by activating the masticatory muscles of the user.

17. The digital corrective device according to Claim 16, wherein the sensing unit is inserted into the main body and includes at least one pressure sensor aligned with the occlusal surface of the user's tooth or arranged between the teeth, and the electrode unit provides the stimulation signal that magnifies the sensory signal of the periodontal ligament nerve and the surrounding gingival nerve beyond a reference value.

18. The digital corrective device according to Claim 17, wherein the digital corrective device is a wake-up device for countering drowsiness by linking with a vehicle system, being worn in the oral cavity of the user according to the user's drowsy driving state, and transmitting the stimulation signal for the activation of the brain.

19. The digital corrective device according to Claim 17, wherein the digital corrective device is a brain activation device for preventing or alleviating Alzheimer's or dementia by transmitting the stimulation signal for activating the user's brain.
